(19) Europäisches Patentamt
European Patent Office
Office européen des brevets

(11) Veröffentlichungsnummer: **0 270 929**
**A2**

(12) # EUROPÄISCHE PATENTANMELDUNG

(21) Anmeldenummer: **87117447.0**

(22) Anmeldetag: **26.11.87**

(51) Int. Cl.4: **C07C 143/822**, C07C 93/14, A61K 31/18

(30) Priorität: **10.12.86 DE 3642105**

(43) Veröffentlichungstag der Anmeldung: **15.06.88 Patentblatt 88/24**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Niewöhner, Ulrich, Dr.**
**Gartenstrasse 3**
**D-5632 Wermelskirchen 3(DE)**
Erfinder: **Hoever, Franz-Peter, Dr.**
**Kunstfelderstrasse 25**
**D-5000 Koeln 80(DE)**
Erfinder: **Lieb, Folker, Dr.**
**Alfred-Kubin-Strasse 1**
**D-5090 Leverkusen(DE)**
Erfinder: **Rosentreter, Ulrich, Dr.**
**Kondorweg 23**
**D-5600 Wuppertal 1(DE)**
Erfinder: **Perzborn, Elisabeth, Dr.**
**Am Terscher Busch 13**
**D-5600 Wuppertal 1(DE)**
Erfinder: **Fiedler, Volker-Bernd, Dr.**
**Lehner Muehle 46**
**D-5000 Leverkusen 3(DE)**
Erfinder: **Seuter, Friedel, Dr.**
**Moospfad 16**
**D-5600 Wuppertal 1(DE)**

(54) **Substituierte Aminomethyl-5,6,7,8-tetrahydronaphtyl-oxy-essigsäuren, neue Zwischenprodukte, Verfahren zu ihrer Herstellung sowie ihre Verwendungin Arzneimitteln.**

(57) Die vorliegende Erfindung betrifft neue substituierte Aminomethyl-5,6,7,8-tetrahydronaphthyl-oxyessigsäuren der allgemeinen Formel I

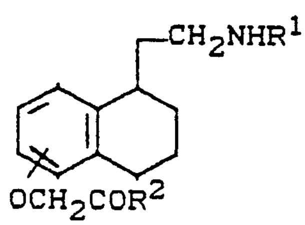

(I)

in welcher $R^1$ und $R^2$ die in der Beschreibung angegebene Bedeutung haben, Verfahren zu ihrer Herstellung sowie ihre Verwendung in Arzneimitteln, insbesondere als Antithrombotika, Antiatherosklerotika und als antiischämische Mittel und neue Zwischenprodukte für ihre Herstellung.

EP 0 270 929 A2

Xerox Copy Centre

BAYER AKTIENGESELLSCHAFT 5090 Leverkusen, Bayerwerk
Konzernverwaltung RP
Patentabteilung KS/by-c

Substituierte Aminomethyl-5,6,7,8-tetrahydronaphthyl-oxyessigsäuren, neue Zwischenprodukte, Verfahren zu ihrer Herstellung sowie ihre Verwendung in Arzneimitteln

Die vorliegende Erfindung betrifft neue substituierte Aminomethyl-5,6,7,8-tetrahydronaphthyl-oxyessigsäuren, Verfahren zu ihrer Herstellung sowie ihre Verwendung in Arzneimitteln, insbesondere als Antithrombotika, Antiatherosklerotika und als antiischämische Mittel und neue Zwischenprodukte für ihre Herstellung.

Thrombose und arteriosklerotische Gefäßveränderungen werden vor allem durch die Wechselwirkung zweier Metaboliten der Arachidonsäure, nämlich durch das Thromboxan $A_2$ ($TXA_2$) und durch das Prostacyclin ($PGI_2$) gesteuert. $TXA_2$ wirkt auf die Blutplättchen aggregierend, und $PGI_2$ hat eine antiaggregierende Wirkung. Darüber hinaus wirkt $TXA_2$ vasokonstriktorisch und $PGI_2$ vasodilatatorisch.

Bei einer Reihe von thrombo-embolischen und ischämischen Erkrankungen führt Hyperaggregabilität der Plättchen bzw.

ein erhöhter Plättchenverbrauch zu einer gesteigerten Thromboxansynthese, so daß das $TXA_2$- und $PGI_2$-Gleichgewicht gestört ist. Es ist deshalb zur Therapie und Prophylaxe von thrombo-embolischen und ischämischen Erkrankungen wünschenswert, die Thromboxanwirkung zu hemmen und damit die protektive Eigenschaften des $PGI_2$ zu erhöhen.

Es wurde nunmehr überraschend gefunden, daß bestimmte substituierte Aminomethyl-5,6,7,8-tetrahydronaphthyl-oxyessigsäuren eine spezifische und starke antagonistische Wirkung bezüglich Thromboxan $A_2$ aufweisen.

Die Erfindung betrifft neue substituierte Aminomethyl-5,6,7,8-tetrahydronaphthyl-oxyessigsäuren der allgemeinen Formel (I)

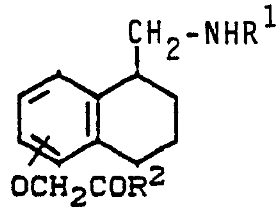

(I)

in welcher

$R^1$ für $SO_2R^3$ steht, wobei

$R^3$ für Aryl oder substituiertes Aryl steht,

$R^2$ für Hydroxy, Alkoxy, Phenoxy, Benzoxy oder $NR^4R^5$ steht wobei

$R^4$ und $R^5$ gleich oder verschieden sind und jeweils für Wasserstoff oder Alkyl stehen oder einer der Reste $R^4$ oder $R^5$ für Benzyl steht,

sowie deren physiologisch verträglichen Salze mit ein- oder zweiwertigen Kationen.

Von besonderem Interesse sind Verbindungen der allgemeinen Formel (I) in welcher

$R^1$ für $SO_2R^3$ steht, wobei

$R^3$ für Phenyl oder Naphthyl steht, welche gegebenenfalls 1, 2 oder 3 gleiche oder verschiedene Substituenten wie Halogen, Cyano, Trifluormethyl und Alkyl mit 1 bis 4 C-Atomen tragen,

$R^2$ für Hydroxyl, Phenoxy, Benzoxy oder Alkoxy mit 1 bis 4 Kohlenstoffatomen steht oder für die Gruppe $NR^4R^5$ steht, wobei $R^4$ und $R^5$ gleich oder verschieden sind und jeweils für Wasserstoff oder Alkyl mit 1 bis 4 Kohlenstoffatomen stehen oder einer der Reste $R^4$ oder $R^5$ für Benzyl steht,

sowie deren physiologisch verträglichen Salze mit ein- oder zweiwertigen Kationen.

Von besonderem Interesse sind Verbindungen der allgemeinen Formel (I) in welcher $R^3$ für durch Fluor oder Chlor substituiertes Phenyl steht.

Die neuen substituierten Aminomethyl-5,6,7,8-tetrahydronaphthalin-oxyessigsäurederivate der Formel (I) können sowohl als Enantiomere, Enantiomerenpaare bzw. bei Vorliegen eines weiteren Asymetriezentrums in einem der Reste als Diastereomerenpaare vorliegen.

Es wurde ferner gefunden, daß man die substituierten Aminomethyl-5,6,7,8-tetrahydronaphthyl-oxyessigsäuren der allgemeinen Formel (I) erhält, wenn man die Amine der allgemeinen Formel (II)

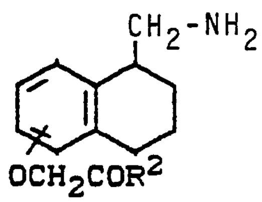

(II)

in denen $R^2$ die oben angegebene Bedeutung hat, in an sich bekannter Art und Weise mit Sulfonsäuren der allgemeinen Formel $R^3SO_3H$, wobei $R^3$ die oben angegebene Bedeutung besitzt, oder deren aktivierte Derivate wie Säurechloride oder aktivierte Ester umsetzt. Im Falle von $R^2 \neq OH$ schließt sich eine Verseifung zu den freien Carbonsäuren an.

Die Aminomethyltetralin-oxyessigsäurederivate II erhält man aus den entsprechenden α,β-ungesättigten Nitrilen der Formel III

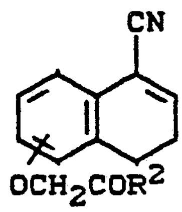

(III)

wobei

$R^2$ die oben angegebene Bedeutung besitzt,

durch katalytische Hydrierung in Gegenwart von $NH_3$. Die Hydrierung kann vorzugsweise nach literaturbekannten Verfahren durchgeführt werden (siehe beispielsweise Rylander, "Catalytic Hydrogenation", Academic Press, Inc., New York, 1967).

Die neuen α,β-ungesättigten Nitrile III erhält man aus Tetralonen der Formel (IV)

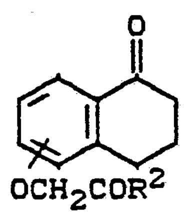

(IV)

wobei

$R^2$ die oben angegebene Bedeutung besitzt,

durch Umsetzung mit Trimethylsilylcyanid, gegebenenfalls in Gegenwart eines Katalysators, wobei die silylierten Cyanhydrine entstehen, die direkt mit Säuren wie z. B. Halogenwasserstoffsäuren, Schwefelsäure, Salpetersäure, Trifluoressigsäure, Methansulfonsäure, p-Toluolsulfonsäure in geeigneten protischen oder aprotischen Lösungsmitteln wie z. B. Methanol, Ethanol, Aceton, Tetrahydrofuran, Essigsäureethylester, Toluol, Benzol oder halogenierten Lösungsmitteln wie Methylenchlorid, Chloroform, Tetrachlorkohlenstoff in den α,ß,-ungesättigten Nitrilen der Formel III umgesetzt werden (vgl. Lit.: D. A. Evans, G.L. Carroll, L.H. Truesdale, J. Org. Chem. <u>39</u>, 914 (1974)).

Statt Trimethylsilylcyanid kann man auch Trimethylsilylchlorid in Gegenwart von Alkalicyanid und Alkalihalogenid in geeigneten Lösungsmitteln wie z. B. Dimethylformamid, Dimethylsulfoxid analog literaturbekannter Verfahren einsetzen (vgl. z. B. T. Hiyama, M. Inoue, K. Saito, Synthesis 1986, 645).

Die Tetralone IV erhält man durch Alkylierung der entsprechenden Hydroxytetralone

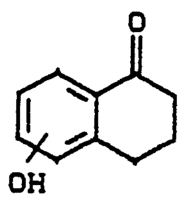

(V)

mit Essigsäurederivaten der allgemeinen Formel VI

$X-CH_2-COR^2$ (VI), in welcher

X eine Abgangsgruppe wie z. B. Cl, Br, I, $SO_2CH_3$,

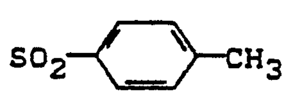

bedeutet und

$R^2$ die oben angegebene Bedeutung besitzt,

nach literaturbekannten Verfahren (z.B. Patai "The Chemistry of the Hydroxyl Group", pt. 1., S. 454-466, Interscience Publishers, New York, 1971; Tetrahedron 30, 1379 (1974)).

Die Hydroxytetralone V sind teilweise literaturbekannt (z.B. J. Org. Chem. 14, S. 366 (1949)), teilweise können sie durch Etherspaltung analog literaturbekannter Verfahren aus den bekannten Methoxytetralonen VII hergestellt werden (z.B. Org. Syntheses, Vo. 51, S. 109; J. Chem. Soc. 1855 (1949)).

Als Beispiele für die als Ausgangsstoffe verwendeten Methoxytretralone VII seien genannt:

5-Methoxy-1-tetralon
6-Methoxy-1-tetralon

Die Synthesesequenzen lassen sich ausgehend von den Methoxytetralonen VII in einem Reaktionsschema wie folgt zusammenfassen:

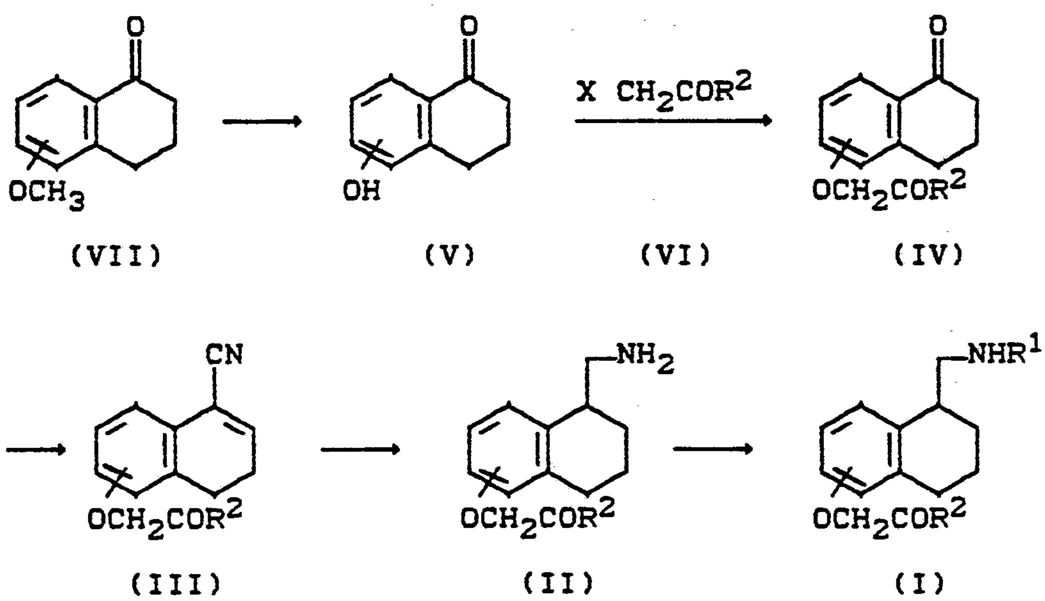


(VII) (V) (VI) (IV)

(III) (II) (I)

wobei

$R^1$, $R^2$ und X die oben angegebenen Bedeutungen haben.

Die Endprodukte I erhält man, indem man die Amine der allgemeinen Formel II mit den entsprechenden Sulfonsäuren $R^3SO_3H$ oder deren aktivierte Derivate wie z.B. Sulfonsäurechloride, Sulfonsäureanhydride oder Sulfonsäureester in Gegenwart eines säurebindenden Mittels wie z.B. Alkali- oder Erdalkalihydroxiden oder Alkali- bzw. Erdalkalicarbonaten oder organischen Basen wie z.B. Triethylamin, Pyridin oder N-Ethylmorpholin umsetzt.

Als Lösungsmittel sind in Abhängigkeit von der Art des eingesetzten Sulfonsäurederivats inerte organische Lösungsmittel wie z.B. Methylenchlorid, Chloroform, Essigester, Tetrahydrofuran, Ether, Dimethylformamid, Pyridin und/oder protische Lösungsmittel wie z.B. Wasser, Methanol, Ethanol geeignet.

Die Amine der allgemeinen Formel II erhält man durch katalytische Hydrierung der α,β-ungesättigten Nitrile III. Als Katalysatoren kommen Metalle wie z.B. Raney-Nickel, Palladium oder Platin in der dem Fachmann bekannten Anwendungsform in Frage. Als Lösemittel können protische Lösungsmittel wie z.B. Methanol, Ethanol und/oder aprotische Lösungsmittel wie z.B. Tetrahydrofuran, Toluol, Dimethylformamid, Methylenchlorid oder Dioxan dienen, denen zweckmäßigerweise flüssiger Ammoniak zugesetzt wird. Die Hydrierung wird bei Drücken zwischen 5 und 300 atm, vorteilhaft zwischen 50 und 150 atm durchgeführt. Die Reaktionstemperatur liegt zwischen 20 und 150°C, vorteilhaft zwischen 30 und 100°C, die Reaktionszeit beträgt 15 Minuten bis 6 Stunden.

Die α,β-ungesättigten Nitrile der allgemeinen Formel III erhält man aus den Tetralonen IV durch Umsetzung mit Trimethylsilylcyanid. Als Katalysatoren dieser Reaktion sind Lewis-Säuren wie z.B. Bortrifluorid-Etherat, Zinkiodid, Zinntetrachlorid, Aluminiumtrichlorid oder organische Basen wie Triethylamin, Pyridin oder auch der Komplex aus 18-Krone-6 und Kaliumcyanid geeignet; die Reaktion kann auch ohne Katalysator durchgeführt werden.

Die Reaktion kann in inerten organischen Lösungsmitteln wie Diethylether, Tetrahydrofuran, Methylenchlorid, Toluol oder auch ohne Lösungsmittel durchgeführt werden.

Die Reaktionstemperatur liegt zwischen 0 und 100° C, bevorzugt zwischen 20 und 60° C.

Die rohen silylierten Cyanhydrine werden durch Behandeln mit einer alkoholischen Halogenwasserstofflösung in die α,β-ungesättigten Nitrile IV überführt. Als Alkohole eignen sich niedere aliphatische Alkohole wie z.B. Methanol oder Ethanol, als Halogenwasserstoffe kommen Chlorwasserstoff und Bromwasserstoff, bevorzugt Chlorwasserstoff in Frage.

Die Reaktionstemperatur liegt zwischen 0 und 50° C, bevorzugt zwischen 0 und 25° C, die Reaktionszeit liegt zwischen 10 Minuten und 6 Stunden.

Statt alkoholischer Halogenwasserstofflösungen sind auch Lösungen anderer Säuren wie z. B. Methansulfonsäure, p-Toluolsulfonsäure, Trifluoressigsäure in protischen oder aprotischen Lösungsmitteln wie z. B. Benzol oder Toluol geeignet.

Die Tetraline der allgemeinen Formel IV erhält man durch Alkylierung der Hydroxytetralone der Formel V

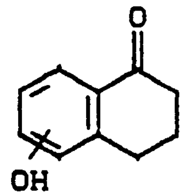

(V)

mit Essigsäurederivaten der allgemeinen Formel VI

$$X\text{-}CH_2COR^2 \qquad (VI)$$

wobei

X und $R^2$ die oben angegebene Bedeutung besitzt.

Die Alkylierung wird zweckmäßig in Gegenwart eines säurebindenden Mittels wie z.B. Alkali- oder Erdalkalihydroxiden oder -carbonaten oder organischen Basen wie z.B. Triethylamin, Pyridin, Diazabicycloundecan in organischen Lösungsmitteln wie Aceton, Butanon, Dimethylformamid, Dimethylsulfoxid, Ethanol, Dioxan, Toluol oder halogenierten Kohlenwasserstoffen wie Methylenchlorid oder Chloroform durchgeführt.

Oft ist es günstig ein Alkalihalogenid wie z.B. Natrium- oder Kaliumiodid und ein wasserbindendes Mittel wie z.B. Molekuklarsieb 3Å zuzusetzen.

Die Reaktionstemperatur liegt in Abhängigkeit vom Lösungsmittel zwischen 50 und 150°, vorzugsweise zwischen 50 und 80° C.

Als Beispiele für die Verbindungen der allgemeinen Formel I seien genannt:

5-(4-Fluorphenylsulfonylaminomethyl)-5,6,7,8-tetrahydro-naphth-1-yl-oxyessigsäure
5-(3-Chlorphenylsulfonylaminomethyl)-5,6,7,8-tetrahydro-naphth-1-yl-oxyessigsäure
5-(4-Methylphenylsulfonylaminomethyl)-5,6,7,8-tetrahydro-naphth-1-yl-oxyessigsäure
5-(4-Trifluormethylphenylsulfonylaminomethyl)-5,6,7,8-tetrahydro-naphth-1-yl-oxyessigsäure
5-(3,4-Dichlorphenylsulfonylaminomethyl)-5,6,7,8-tetrahydro-naphth-1-yl-oxyessigsäure
5-(3-Chlorphenylsulfonylaminomethyl)-5,6,7,8-tetrahydro-naphth-2-yl-oxyessigsäure
5-(4-Methylphenylsulfonylaminomethyl)-5,6,7,8-tetrahydro-naphth-2-yl-oxyessigsäure
5-(4-Trifluormethylphenylsulfonylaminomethyl)-5,6,7,8-tetrahydro-naphth-2-yl-oxyessigsäure

Als Zubereitungsformen kommen die üblichen galenischen Applikationsformen in Frage, beispielsweise Cremes, Tabletten, Pillen, Kapseln, Suppositorien, Emulsionen, Infusions- und Injektionslösungen. Diese Zubereitungsformen werden nach an sich bekannten Methoden hergestellt unter Verwendung üblicher Hilfs- und Trägerstoffe.

Der Einsatz der so hergestellten Arzneimittel erfolgt je nach Bedarf z.B. durch lokale, parenterale oder orale Verabreichung.

Besonders geeignet sind Formulierungen, die die erfindungsgemäßen Verbindungen in Konzentrationen von etwa 0,1

bis 10 Gew.-% enthalten. Besonders bevorzugt sind wäßrige Lösungen, die gegebenenfalls auf einen pH-Wert von 6 bis 8 gepuffert sind.

Die Dosierung der substituierten Aminomethyl-5,6,7,8-tetrahydro-naphthyl-oxyessigsäurederivate in den erfindungsgemäßen Arzneimitteln liegt vorzugsweise in einem Bereich von 0,05 bis 100 mg/kg, insbesondere von 0,1 bis 20 mg/kg Körpergewicht.

Die in den erfindungsgemäßen Arzneimitteln enthaltenen substituierten Aminomethyl-5,6,7,8-tetrahydronaphthyl-oxyessigsäuren sind als Thromboxanantagonisten und Plättchenaggregationshemmer zur Verhütung und Behandlung von Thrombosen, Thromboembolien, ischämischen Erkrankungen, als Antiasthmatika und als Antiallergika geeignet.

Methodik

Thrombozytenaggregationshemmung in vitro

Für die in vitro-Bestimmung der thrombozytenaggregationshemmenden Wirkung wird Blut von gesunden Spendern, die mindestens 14 Tage lang kein Medikament eingenommen hatten, verwendet. Das Blut wird in 3,8 %iger Natriumcitratlösung aufgenommen. Plättchenreiches Plasma (PRP) wird durch 20 Min. lange Zentrifugation bei 150 g bei Raumtemperatur gewonnen (Jürgens/Beller: Klinische Methoden der Blutgerinnungsanalyse; Thieme Verlag, Stuttgart

1959). Die Plättchenaggregation wird nach der turbidometrischen Methode (Born, G.V.R.: J. Physiol. 162, 67, 1962) im Aggregometer bei 37° C bestimmt. Hierzu wird PRP mit der Prüfsubstanz bei 37° C inkubiert und anschließend die Aggregation durch Zugabe einer Kollagensuspension ausgelöst. Für die in-vitro-Versuche wird die minimal effektive Wirkstoffkonzentration (MEK) angegeben, die in den entsprechenden PRP-Proben die Thrombozytenaggregation hemmt.

Thrombozytenaggregationshemmung ex vivo

Für die ex vivo-Untersuchungen wird den Tieren die Wirksubstanz in einer Tylosesuspension oral verabreicht. Nach 90 Minuten werden die Tiere entblutet und das PRP mittels Zentrifugation gewonnen. Die Messung der Aggregationshemmung erfolgt analog dem Verfahren, welches für die in vitro-Versuche beschrieben ist; jedoch ohne Vorinkubation der Proben.

In der Tabelle sind Ergebnisse der Kollagen induzierten Thrombozytenaggregation einiger Beispiele aufgeführt.

| Beispiel Nr. | Hemmung der Thrombozytenaggregation (in vitro) Grenzkonzentration [mg/l] |
|---|---|
| 9 | 0,3 |
| 10 | 0,3 |
| 15 | 0,01 |
| 16 | 0,1 |
| 17 | 1 |

Beispiel 1

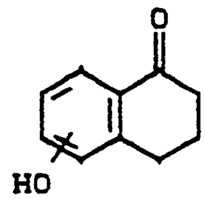

6-Hydroxy-1-tetralon

2 Mol 6-Methoxy-1-tetralon werden in 2 l 48 %iger wäßriger Bromwasserstoffsäure 3 h auf 125°C erhitzt. Beim Abkühlen fällt das Produkt aus und wird abgesaugt und getrocknet. Bei Bedarf kann aus Wasser umkristallisiert werden.
Ausbeute 86,4 % der Theorie
Fp.: 155-57°C

Beispiel 2

5-Hydroxy-1-tetralon

1 Mol 5-Methoxy-1-tetralon werden in 5 l Methylenchlorid gelöst und auf -78°C gekühlt. Zu dieser Lösung werden 500 ml Bortribromid in 2,3 l Methylenchlorid zugetropft. Es wird 30 Min. bei -78°C, dann 2,5 Std. bei 0°C gerührt, wieder auf -78°C gekühlt und langsam 3 l Methanol zugetropft. Es wird eingedampft, der Rückstand in Methylenchlorid aufgenommen und 3 mal mit 10 %iger Natriumhydroxidlösung extrahiert. Die vereinigten Natriumhydroxidlösungen werden mit konzentrierter Salzsäure angesäuert und 3 mal mit Methylenchlorid ausgeschüttelt. Nach Trocknen über Natriumsulfat wird eingedampft. Der Rückstand ist sauberes Produkt und braucht nicht umkristallisiert zu werden. Der Schmelzpunkt liegt bei 210-215°C.

Beispiel 3

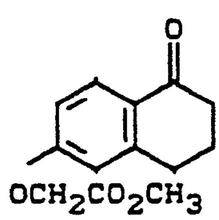

5-Oxo-5,6,7,8-tetrahydro-naphth-2-yl-oxyessigsäuremethylester

1 Mol 6-Hydroxy-1-tetralon, 1,2 Mol Chloressigsäuremethylester, 2 Mol Kaliumcarbonat und 0,1 Mol Kaliumiodid werden in 2 l Butanon 6 h am Rückfluß gekocht. Es wird abfiltriert, mit Aceton nachgewaschen und eingedampft. Der Rückstand wird in Methylenchlorid aufgenommen, 3 mal mit 0,5 n NaOH-Lösung gewaschen, über Natriumsulfat getrocknet und eingedampft.

Ausbeute: 92 % der Theorie

Fp.: 116-118°C

In analoger Weise zu Beispiel 3 wurde hergestellt:

Beispiel 4

5-Oxo-5,6,7,8-tetrahydro-naphth-1-yl-oxyessigsäuremethylester

Ausbeute: 77 % der Theorie

Fp.: 84 - 85° (Ligroin)

Beispiel 5

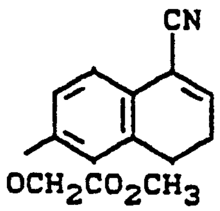

5-Cyano-7,8-dihydro-naphth-2-yl-oxyessigsäuremethylester

0,1 Mol 5-Oxo-5,6,7,8-tetrahydro-naphth-2-yl-oxyessigsäuremethylester und 0,15 Mol Trimethylsilylcyanid werden mit 2 bis 3 Tropfen $BF_3$-Etheratlösung versetzt, 30 Minuten bei Raumtemperatur, dann 1 Stunde bei 50° C gerührt. Zu dieser Lösung werden bei Raumtemperatur 250 ml einer gesättigten methanolischen Chlorwasserstofflösung gegeben und 1 Stunde gerührt. Ein Teil des Produktes fällt aus und wird abfiltriert. Eine weitere Charge erhält man durch Eindampfen der Mutterlauge, Aufnehmen des Rückstands in $CH_2Cl_2$, zweimaliges Ausschütteln mit 0,5 n NaOH, Trocknen über $Na_2SO_4$, Eindampfen und Umkristallisation aus wenig Methanol oder Diisopropylether.
Ausbeute: 71 % der Theorie
Fp.: 95° C

In analoger Weise zu Beispiel 5 wurde hergestellt:

Beispiel 6

5-Cyano-7,8-dihydro-naphth-1-yl-oxyessigsäuremethylester

Ausbeute: 63 % der Theorie
Fp.: 112° C

Beispiel 7

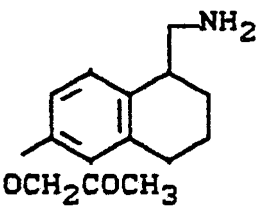

5-Aminomethyl-5,6,7,8-tetrahydro-naphth-2-yl-oexessigsäureamid

0,25 Mol des α,β-ungesättigten Nitrils aus Beispiel 5 werden in 600 ml Methanol und 185 ml flüssigem Ammoniak gelöst. Nach Zugabe von 22 g Methanol-feuchtem Raney-Nickel wird 3 Stunden bei 70° C und 100 atm Wasserstoff hydriert. Es wird kurz zum Rückfluß erhitzt, der Katalysator heiß abfiltriert und dann eingedampft. Das Produkt kann direkt weiterverarbeitet werden (siehe Beispiel 9) oder aus Diglyme als Hydrochlorid gefällt werden.
Ausbeute: 96,7 % der Theorie
$^{1}$H-NMR (Hydrochlorid in $CD_3OD$): δ = 1,75 (m, 4H); 2,78 (m, 2H); 3,1 (m, 2H); 3,15 (m, 1H); 4,45 (s, 2H); 6,8 (m, 2H); 7,17 (m, 1H)

In analoger Weise zu Beispiel 7 wurde hergestellt:

Beispiel 8

5-Aminomethyl-5,6,7,8-tetrahydro-naphth-1-yl-oxyessigsäureamid

Ausbeute: 94,2 % der Theorie

$^1$H-NMR (Hydrochlorid in $CD_3OD$): δ = 1,75 (m, 4H); 2,8 (m, 2H); 3,1 (m, 2H); 3,25 (m, 1H); 4,45 (s, 2H); 6,7 bis 7,0 (m, 3H)

Beispiel 9

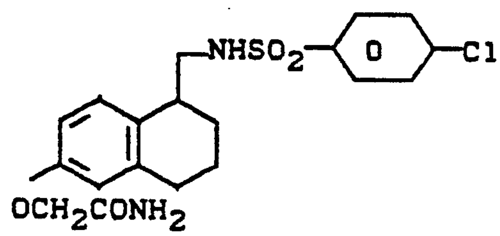

5-(4-Chlorphenylsulfonylaminomethyl)-5,6,7,8-tetrahydro-naphth-2-yl-oxyessigsäureamid

0,1 Mol 5-Aminomethyl-5,6,7,8-tetrahydro-naphth-2-yl-oxyessigsäureamid (Beispiel 7), 0,11 Mol 4-Chlorbenzol-sulfonsäurechlorid und 0,11 Mol Triethylamin werden in 200 ml THF 3 Stunden bei Raumtemperatur gerührt. Es wird abfiltriert, eingedampft, in $CH_2Cl_2$ aufgenommen und mit 1n HCl, gesättigter $NaHCO_3$, 1n HCl und gesättigter NaCl Lösung gewaschen, über $Na_2SO_4$ getrocknet und eingedampft. Der Rückstand wird durch Flashchromatographie über Kieselgel mit $CH_2Cl_2$/MeOH 5:1 gereinigt.
Ausbeute: 68 % der Theorie
$^1$H-NMR ($CDCl_3$): δ = 1,75 (m, 4H); 2,7 (m, 2H); 2,9 (q, 1H); 3,15 (d, 2H); 4,4 (s, 2H); 5,0 (m, 1H); 5,95 (m, 1H); 6,5 (m, 1H); 6,65 (m, 2H); 7,0 (d, 2H); 7,45 (d, 2H); 7,8 (d, 2H)

In analoger Weise zu Beispiel 9 wurden hergestellt:

Beispiel 10

5-(4-Fluorphenylsulfonylaminomethyl)-5,6,7,8-tetrahydro-naphth-2-yl-oxyessigsäureamid
Ausbeute: 77,8 % der Theorie

$^1$H-NMR ($CD_3OD$): δ = 1,7 (m, 4H); 2,7 (m, 2H); 3,0 (m, 1H); 3,3 (m, 2H); 4,45 (s, 2H); 6,7 (m, 2H); 7,0 (d, 1H); 7,7 bis 8,3 (m, 4H)

Beispiel 11

5-(3,4-Dichlorphenylsulfonylaminomethyl)-5,6,7,8-tetrahydro-naphth-2-yl-oxyessigsäureamid
Ausbeute: 80,2 % der Theorie

$^1$H-NMR ($CDCl_3$): δ = 1,7 (m, 4H); 2,7 (m, 2H); 2,9 (q, 1H); 3,15 (m, 2H); 4,4 (1, 2H); 5,2 (m, 1H); 6,0 (m, 1H); 6,6 (m, 1H); 6,65 (m, 2H); 7,0 (d, 1H); 7,3 bis 7,9 (m, 3H)

Beispiel 12

5-Phenylsulfonylaminomethyl-5,6,7,8-tetrahydro-naphth-2-yl-oxyessigsäureamid
Ausbeute: 73 % der Theorie

$^1$H-NMR ($CDCl_3$): δ = 1,75 (m, 4H); 2,7 (m, 2H); 2,95 (q, 1H); 3,1 (m, 2H); 4,4 (s, 2H); 5,2 (m, 1H); 5,95 (m, 1H); 6,6 (m, 1H); 6,65 (m, 2H); 7,0 (d, 1H); 7,4 bis 7,7 (m, 3H); 7,8 bis 8,05 (m, 2H)

**Beispiel 13**

5-(4-Chlorphenylsulfonylaminomethyl)-5,6,7,8-tetrahydro-naphth-1-yl-oxyessigsäureamid
Ausbeute: 53 % der Theorie
Fp.: 174°C (nach Chromatographie, nicht umkristallisiert)
$^1$H-NMR ($CCD_3OD$): $\delta$ = 1.7 (m, 4H); 2.6-3.1 (m, 5H); 4.45 (s, 2H); 6.7 (m, 2H); 7.05 (m, 1H); 7.6 (m, 2H); 7.8 (m, 2H)

**Beispiel 14**

5-Phenylsulfonylaminomethyl-5,6,7,8-tetrahydro-naphth-1-yl-oxyessigsäureamid
Ausbeute: 57 % der Theorie

$^1$H-NMR ($CDCl_3$): $\delta$ = 1,7 (m, 4H); 2,75 (m, 2H); 2,9 (q, 1H), 3,15 (m, 2H); 4,4 (s, 2H); 5,1 (m, 1H); 6,0 (m, 1H); 6,6 (m, 1H)=; 6,9 (m, 1H); 7,3 (m, 2H); 7,4 bis 7,7 (m, 3H); 7,8 bis 8,0 (m, 2H)

**Beispiel 15**

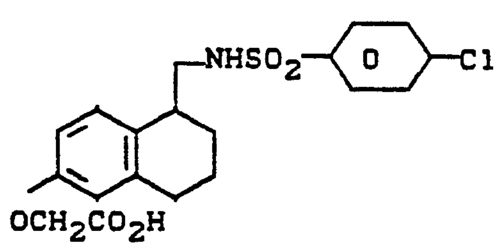

5-(4-Chlorphenylsulfonylaminomethyl)-5,6,7,8-tetrahydro-naphth-2-yl-oxyessigsäure

0,01 Mol 5-(4-Chlorphenylsulfonylaminomethyl)-5,6,7,8-tetrahydro-naphth-2-yl-oxyessigsäure werden mit 0,025 Mol KOH in 32 ml $H_2O$ und 100 ml Methanol 6 h am Rückfluß gekocht. Das Methanol wird abgedampft, die wäßrige Phase zweimal mit Methylenchlorid ausgeschüttelt und mit konzentrierter Salzsäure auf pH 1 gebracht und 2 mal mit Methylenchlorid ausgeschüttelt. Nach Trocknen über $Na_2SO_4$ wird eingedampft, wobei die Produkte sauber als Feststoffe anfallen.
Ausbeute: 84 % der Theorie
Fp.: 55 bis 60°C Schaum, nicht umkristallisiert
IR (KBr): 1740 $cm^{-1}$ Carbonylbande

In analoger Weise zu Beispiel 15 wurden hergestellt:

Beispiel 16

5-(4-Fluorphenylsulfonylaminomethyl)-5,6,7,8-tetrahydro-naphth-2-yl-oxyessigsäure
Ausbeute: 48,5 % der Theorie
IR (KBr): 1740 $cm^{-1}$ Carbonylbande

Beispiel 17

5-(3,4-Dichlorphenylsulfonylaminomethyl)-5,6,7,8-tetrahydro-naphth-2-yl-oxyessigsäure
Ausbeute: 61 % der Theorie
IR (KBr): 1735 $cm^{-1}$ (Carbonylbande)

Beispiel 18

5-Phenylsulfonylaminomethyl-5,6,7,8-tetrahydro-naphth-2-yl-oxyessigsäure
Ausbeute: 88 % der Theorie
IR (KBr): 1740 $cm^{-1}$ (Carbonylbande)

Beispiel 19

5-(4-Chlorphenylsulfonylaminomethyl)-5,6,7,8-tetrahydro-naphth-1-yl-oxyessigsäure
Ausbeute: 81 % der Theorie, Fp.: 170°C (Schaum nach Chromatographie nicht umkristallisiert)
IR (KBr): 1735 $cm^{-1}$ (Carbonylbande)

Beispiel 20

5-Phenylsulfonylaminomethyl-5,6,7,8-tetrahydro-naphth-1-yl-oxyessigsäure
Ausbeute: 81 % der Theorie
IR (KBr): 1740 $cm^{-1}$ (Carbonylbande)

Beispiel 21

5-(4-Fluorphenylsulfonylaminomethyl)-5,6,7,8-tetrahydro-naphth-1-yl-oxyessigsäure
Ausbeute: 78 % der Theorie
IR(KBr): 1738 $cm^{-1}$ (Carbonylbande)
$^1$H-NMR ($CD_3OD$): $\delta$ = 1.6-1.95 (m, 4H); 2.55-3.05 (m, 5H); 4.62 (s, 2H); 6.55-6.8 (m, 2H); 7.0 (m, 1H); 7.3 (m, 2H); 7.9 (m, 2H)

Beispiel 22

5-(4-Fluorphenylsulfonylaminomethyl)-5,6,7,8-tetrahydro-naphth-1-yl oxyacetamid

Ausbeute: 49 % der Theorie

Fp: 86°C (Schaum nach Chromatographie, nicht umkristallisiert)

Patentansprüche

1. Aminomethyl-5,6,7,8-tetrahydronaphthyl-oxyessigsäuren der allgemeinen Formel (I)

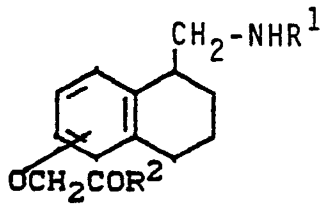

(I)

in welcher

$R^1$ für $SO_2R^3$ steht, wobei

$R^3$ für Aryl oder substituiertes Aryl steht,

$R^2$ für Hydroxy, Alkoxy, Phenoxy, Benzoxy oder $NR^4R^5$ steht wobei

$R^4$ und $R^5$ gleich oder verschieden sind und jeweils für Wasserstoff oder Alkyl stehen oder einer der Reste $R^4$ oder $R^5$ für Benzyl steht,

sowie deren physiologisch verträglichen Salze mit ein-oder zweiwertigen Kationen.

2. Verbindungen der allgemeinen Formel (I) gemäß Anspruch 1, in welcher

$R^1$ für $SO_2R_3$ steht, wobei

$R^3$ für Phenyl oder Naphthyl steht, welche gegebenenfalls 1, 2 oder 3 gleiche oder verschiedene Substituenten wie Halogen, Cyano, Trifluormethyl und Alkyl mit 1 bis 4 C-Atomen tragen,

$R^2$ für Hydroxyl, Phenoxy, Benzoxy oder Alkoxy mit 1 bis 4 Kohlenstoffatomen steht oder für die Gruppe $NR^4R^5$ steht, wobei $R^4$ und $R^5$ gleich oder verschieden sind und jeweils für Wasserstoff oder Alkyl mit 1 bis 4 Kohlenstoffatomen stehen oder einer der Reste $R^4$ oder $R^5$ für Benzyl steht,

sowie deren physiologisch verträglichen Salze mit ein- oder zweiwertigen Kationen.

3. Verbindungen der allgemeinen Formel (I) gemäß Anspruch 1, in welcher $R^3$ für mit Fluor oder Halogen substituiertes Phenyl steht.

4. Verfahren zur Herstellung von Aminomethyl-5,6,7,8-tetrahydronaphthyl-oxyessigsäuren der allgemeinen Formel (I)

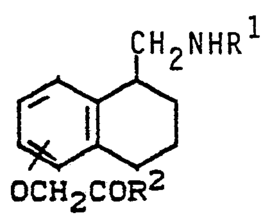

(I)

in welcher

$R^1$ für $SO_2R^3$ steht, wobei

$R^3$ für Aryl oder substituiertes Aryl steht,

$R^2$ für Hydroxy, Alkoxy, Phenoxy, Benzoxy oder $NR^4R^5$ steht wobei

$R^4$ und $R^5$ gleich oder verschieden sind und jeweils für Wasserstoff oder Alkyl stehen oder einer der Reste $R^4$ oder $R^5$ für Benzyl steht,

dadurch gekennzeichnet, daß man Amine der allgemeinen Formel (II)

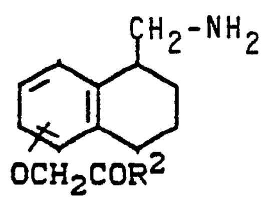

(II)

in welcher

$R^2$ die oben angegebene Bedeutung hat,

mit Säuren der allgemeinen Formel $R^3SO_3H$,

worin

$R^3$ die oben angegebene Bedeutung besitzt,

oder mit deren aktivierten Derivaten wie Säurechloriden, -anhydriden oder aktivierten Estern umsetzt, wobei im Falle für $R^2 \neq OH$ gegebenenfalls eine Verseifung zur freien Carbonsäure durchgeführt wird.

5. Verfahren zur Herstellung von Aminen der allgemeinen Formel (II) gemäß Anspruch 4, dadurch gekennzeichnet, daß man die α,β-ungesättigten Nitrile der allgemeinen Formel (III)

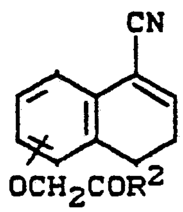

(III)

in welcher

$R^2$ die oben angegebene Bedeutung besitzt,

durch katalytische Hydrierung in Gegenwart von Ammoniak bei erhöhtem Druck reduziert.

6. Verfahren zur Herstellung der α,β-ungesättigten Nitrile III, dadurch gekennzeichnet, daß man die Tetralone der allgemeinen Formel (IV)

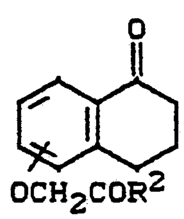

(IV)

in denen

$R^2$ die oben angegebene Bedeutung besitzt

zunächst mit Trimethylsilylcyanid in die silylierten Cyanhydrine überführt, die dann durch Behandeln mit alkokolischer Halogenwasserstofflösung in die α,β-ungesättigten Nitrile III überführt werden.

7. Verbindungen der allgemeinen Formel (II)

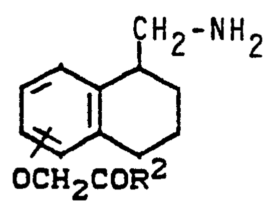

(II)

in welcher

$R^2$ die in Anspruch 1 angegebene Bedeutung hat.

8. Verwendung von Verbindungen der allgemeinen Formel (II) gemäß Anspruch 6 zur Herstellung von Verbindungen der allgemeinen Formel (I) gemäß Anspruch 1.

9. Arzneimittel enthaltend eine Verbindung der allgemeinen Formel (I) gemäß Anspruch 1.

10. Verfahren zur Herstellung von Arzneimitteln, dadurch gekennzeichnet, daß man Verbindungen der allgemeinen Formel (I) gemäß Anspruch 1, gegebenenfalls unter Verwendung von inerten Hilfs- und Trägerstoffen in eine geeignete Applikationsform überführt.

11. Verwendung von Verbindungen der allgemeinen Formel (I) gemäß Anspruch 1 bei der Bekämpfung von Erkrankungen.

12. Verwendung von Verbindungen der allgemeinen Formel (I) gemäß Anspruch 1 bei der Herstellung von Arzneimitteln zur Bekämpfung von thrombo-embolischen, ischämischen und arteriosklerotischen Erkrankungen.